Europäisches Patentamt

European Patent Office

Office européen des brevets

(11) Publication number: **0 160 517**
**B1**

# EUROPEAN PATENT SPECIFICATION

(45) Date of publication of patent specification: **10.08.88**

(51) Int. Cl.⁴: **A 61 F 13/16**

(21) Application number: **85302904.9**

(22) Date of filing: **25.04.85**

(54) Pantiliner.

(30) Priority: **21.06.84 US 623273**
**30.04.84 US 605713**

(43) Date of publication of application:
**06.11.85 Bulletin 85/45**

(45) Publication of the grant of the patent:
**10.08.88 Bulletin 88/32**

(84) Designated Contracting States:
**AT BE CH DE FR IT LI LU NL SE**

(56) References cited:
**EP-A-0 104 906**
**DE-A-2 658 604**
**DE-A-2 806 401**
**DE-A-3 219 234**

(73) Proprietor: **THE PROCTER & GAMBLE COMPANY**
**One Procter & Gamble Plaza**
**Cincinnati Ohio 45202 (US)**

(72) Inventor: **Osborn III, Thomas Ward**
**400 Deanview Drive**
**Cincinnati Ohio 45224 (US)**

(74) Representative: **Gibson, Tony Nicholas et al**
**Procter & Gamble (NTC) Limited Whitley Road**
**Longbenton Newcastle upon Tyne NE12 9TS**
**(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

BACKGROUND OF THE INVENTION
Field of the invention
This invention relates to an absorbent device which is a species of sanitary napkin commonly denominated a pantiliner.

Background art
Sanitary napkins are used by women principally during their menstrual periods to receive and contain menses and other vaginal discharges to protect their garments from soiling. While early versions of sanitary napkins required the use of various specialized belts and supporters, modern designs provide for the adhesive attachment of the sanitary napkin directly to the crotch region of the user's undergarment, normally her panty. Modern sanitary napkins can be efficient and effective at accomplishing their intended purposes.

Many women in modern society have developed the habit of wearing an absorbent device between their menstrual periods to protect their clothing from any small quantity of vaginal discharges then present. Sanitary napkins are frequently used for this purpose. While such use is efficient, conventional sanitary napkins are usually rather bulky (because they are designed to contain relatively large volumes of fluid) and their use is less comfortable to the wearer than the use of an undergarment alone.

Devices which are less bulky, and consequently more comfortable to use, than conventional sanitary napkins have appeared in the consumer market. These devices are usually intended to be affixed to the crotch region of the user's undergarment during times between her menstrual periods, during times when menstrual flow is light, and as supplemental protection for other catamenial products. They have, because of their use and relatively small size, been called pantiliners or, in some literature, panty liners.

Frequently, pantiliners are scaled down versions of conventional sanitary napkins. The present invention departs from this practice by providing a unique, exemplary device designed specifically to be a pantiliner.

Morse, in U.S. Patent 3,371,667 issued March 5, 1968, describes a sanitary napkin comprising a conventional wrapper, a "functional, highly porous resilient element immediately adjacent the surface of the absorbent product which is to be placed against the body and which element serves to entrap highly viscous, mucoid and gelatinous constituents of the body fluids", and a "highly absorbent, relatively dense core". The resilient element is of low density and can comprise synthetic fibers such as polyamides and polyesters. It is said to be essential that these fibers be stabilized. The interstitial walls of the low density web are rendered hydrophilic by treatment with a wetting agent.

Harwood et al, in U.S. Patent 3,029,817 issued April 17, 1962, describe a sanitary napkin comprising an absorbent core or batt and a control element. The batt is "of a conventional highly absorbant material of the type used for absorbent bandages", and the control element "preferably consists of fluid repellent fibers such as synthetic fibers, but certain other fibers which to some extent are wettable may be employed". It is preferred that the fibers in the control element "range in length from about one inch upwardly".

Sanitary napkins comprising an overwrap and an absorbent element having a degree of resiliency are disclosed in DE—A—2658604 and DE—A—3129234.

DE—A—2658604 discloses an absorbent nether garment liner adopted to be adhesively secured to the garment and comprising an absorbent layer in combination with a substnatially inelastic body fluid-impervious layer adhered to one surface of the absorbent layer. The latter comprises hydrophilic absorbent fibers bonded with an elastic binder or a polymeric hydrophilic urethane foam. The absorbent layer thus has a degree of resilience and elasticity under tensile stress and attachment of the absorbent layer to the fluid impervious layer takes place while the former is under tension. Slow recovery of the absorbent layer to its original relaxed length causes the liner to assume an arcuate non planar configuration adapted to fit the abdominal contours of a wearer.

DE—A—3129234 discloses a sanitary napkin comprising an absorbent pad in which a microfibrous thermoplastic web is disposed as a wicking layer intermediate two layers of absorbent materials, the assembly being enclosed by a fluid-pervious overwrap. A fluid impervious sheet is preferably provided between the outwardly facing surface of the absorbent pad and the overwrap. The absorbent layers preferably comprise a mixture of cellulosic fibers and fusible fibers to provide a degree of resilience to the absorbent body.

Summary of the invention
According to the present invention there is provided a pantiliner comprising a means for containing vaginal discharges and a porous overwrap enclosing said means, said pantiliner optionally being provided with adhesive attachment means for securement to the undergarment of the wearer, said means for containing vaginal discharges comprising a batt of hydrophilic fibers bonded together so as to provide resiliency, characterised in that said batt consists solely of bonded synthetic fibers, the surfaces of said fibers being hydrophilic, said bonded fibrous batt having a density of from 0.01 g to 0.1 g/ml and being capable of recovery to 80% of its original volume after release from a compression force that reduces its volume to 20% of its original volume, said resilience being essentially unaffected by moisture.

Brief description of the drawings
Figure 1 is a plan view of a pantiliner of this invention.

Figure 2 is a cross sectional view of the pantiliner shown in Figure 1 taken along line 2—2 of Figure 1.

Figure 3 is a cross sectional view of an alternate embodiment of a pantiliner of this invention as if taken along line 2—2 of Figure 1.

Figure 4 is a cross sectional view of still another embodiment of a pantiliner of this invention as if taken along line 2—2 of Figure 1.

Figure 5 is a plan view of an alternate embodiment of the pantiliner of this invention.

Figure 6 is a cross sectional view of the pantiliner shown in Figure 5 taken along line 6—6 of Figure 5.

In the drawings the thickness of certain materials have been exaggerated for clarity. In the various figures, reference numerals are used consistently to refer to identical or equivalent elements.

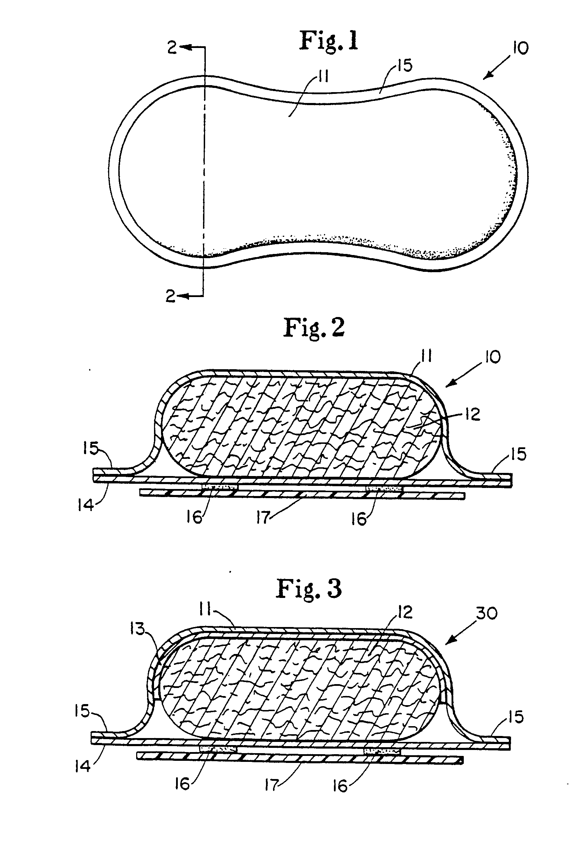

A preferred embodiment of the present invention, pantiliner 10, is shown in plan view in Figure 1 and in cross sectional view in Figure 2. Pantiliner 10 consists of the two necessary components: an overwrap (exemplified by top overwrap 11 and a bottom overwrap 14) and resilient element 12.

In Figure 2 the overwrap is shown in two sections, top overwrap 11 and bottom overwrap 14, rather than as a single section because of the ease of constructing pantiliner 10 when the overwrap is in two parts. It is to be understood that the precise number of sections joined to form the overwrap is immaterial. In fact, an overwrap constructed of a single section of material is illustrated and discussed infra.

The overwrap is any soft, smooth, compliant, porous material which will be comfortable against human skin and through which vaginal discharges will tend to pass. Those skilled in the art can readily select woven and non-woven materials useful for this purpose. In general, porous materials used as topsheets for disposable diapeters or as coverings for conventional sanitary napkins can be used in the present invention.

Preferred overwraps include formed thermoplastic films such as those described with the particularity in U.S. Patent 4,324,246 issued to Mulane and Smith on April 13, 1982 as well as in U.S. Patent 4,342,314 issued to Radel and Thompson on August 3, 1982. Other overwraps useful in the present invention are described in U.S. Patent 4,341,217 and in DE—A—2806401.

The second necessary component of the present invention is the resilient element indicated by reference numeral 12 in Figure 2. The resilient element must be compressible, conformable, and resilient. That is to say, the resilient element must possess such physical properties that forces applied to it by the action of the body of the pantiliner user will readily cause the resilient element to bend and to compress and to conform to the space available for it as the pantiliner is held adjacent the user's body.

The resilient element comprises a mass or batt of synthetic polymeric fibers, which mass or batt must, without the application of external forces, return to essentially its original size and shape after deforming forces are removed. For the purposes of the present invention the resilient element must possess such resilience that it will recover at least 80% of its original volume after it is compressed to 20% of its original volume and the compressing forces are removed. Its resilience must be essentially unaffected by the presence of moisture such as the moisture in vaginal discharges; that is to say, the resilient element must be essentially moisture insensitive.

The resilient element must be of a relatively low density so that it has sufficient void volume to contain practical quantities of vaginal discharges. Low density can also help to ensure that the resilient element is readily deformable under the influence of the user's body thereby exhibiting comfort attributes. For the purposes of the present invention the density of the resilient element should be from 0.01 to 0.1 gram per cubic centimeter.

The surfaces of the interstices of the resilient element must be hydrophilic. More generally, the resilient element must comprise a material which is wetted by the fluids in question. Vaginal discharges and other bodily fluids are primarily aqueous solutions and suspensions; surfaces which are wetted by these fluids can be broadly described as hydrophilic. As used in this specification, the term "hydrophilic" describes surfaces which are wetted by the fluid in question. Thus, the resilient element must be hydrophilic.

The state of the art respecting wetting of materials allows definition of hydrophilicity (and wetting) in terms of contact angles and the surface tensions of the fluids and solids involved. This is discussed in detail in The American Chemical Society publication entitled *Contact Angle, Wetability, And Adhesion* edited by Robert F. Gould, and copyrighted in 1964.

A surface is said to be wetted by a fluid either when the contact angle between the fluid and the surface is less than 90° or when the fluid will tend to spread spontaneously across the surface; both conditions normally coexist.

Vaginal discharges normally have a surface tension of from 35 to 60 dynes per centimeter. They will have contact angles of less than 90° and will tend to spread spontaneously across a solid surface which has a critical surface tension value greater than the fluid surface tension. Since the surface tension of water is normally higher than that of vaginal discharges, any solid which is wetted by water (i.e. which is literally hydrophilic in the precise, limited meaning of the word) is also usually wetted by vaginal discharges.

The materials used in the resilient element can achieve hydrophilicity by any convenient means. For example, the material itself can be intrinsically hydrophilic, although, as discussed infra, this circumstance is relatively rare for materials useful in the resilient element. The surfaces of the resilient element can be rendered hydrophilic by treatment with a surfactant, such as a nonionic or an anionic surfactant, as by spraying the material with a surfactant or dipping the material into the surfactant.

A resilient element possessing the requisite properties can be obtained most readily through the use of a batt of synthetic fibers bonded one to another at a significant number of their points of contact.

Synthetic fibers useful in the present invention include those made of cellulose acetate, polyvinyl chloride, polyvinylidene chloride, acrylic resins, polyvinyl acetates, non-soluble polyvinyl alcohols, polyethylenes, polypropylenes, polyamides, and, preferably, polyesters. Preferred polyester fibers are of from 0.44 to 1.65 Tex and have a length of from 2 to 8 centimeters.

As indicated supra, resiliency of the resilient element can frequently be enhanced if the fibers are bonded together at their points of contact. thermal bonding can be used or, preferably, adhesives, such as latex adhesives, can be used to bond the synthetic fibers one to another.

As discussed supra, the surfaces of the interstices of the resilient element, and, in turn, the surfaces of the fibers, must be hydrophilic. Hydrophilicity can be achieved by selecting fibers which are inherently hydrophilic. The problem with achieving hydrophilicity by this method is that hydrophilic fibers generally lose their resiliency in the presence of moisture. Preferably, then, synthetic fibers such as polyester are used and are treated with surfactant as discussed supra to render the surfaces hydrophilic.

Suitable surfactants include nonionic surfactants such as Brij 76 manufactured by ICI Americas, Inc. of Wilmington, Delaware and the various materials sold under the Pegosperse trademark by Glyco Chemicals, Inc. of Greenwich, Connecticut. Anionic surfactants can also be used. Surfactants are applied to the fibers at a level of from 0.2 to 1 gram per square meter of resilient element.

Referring again to Figures 1 and 2 which illustrate a preferred embodiment of the present invention, top overwrap 11 and bottom overwrap 14 are placed on either side of resilient element 12 and are sealed about the periphery of pantiliner 10 with seal 15. Seal 15 can be achieved by mechanical crimping, thermal welding, ultrasonic welding, use of adhesive, etc.

In the embodiment illustrated in Figure 2, pantiliner 10 is provided with optional adhesive fastening means 16. In Figure 2 adhesive fastening means 16 are illustrated as two narrow strips running essentially the entire length of pantiliner 10. This arrangement is selected for convenience; those skilled in the art can readily select a different pattern for the adhesive attachment means.

The purpose of the adhesive attachment means is, of course, to secure the pantiliner in the crotch of the user's undergarment. Any adhesive or glue used with sanitary napkins for such purposes can be used with this invention. Pressure sensitive adhesives are preferred. Suitable adhesives include Century A-305IV manufactured by Century Adhesive Corporation and Instant Lok 34-2823 manufactured by National Starch Company.

Other means for physically securing the pantiliner in the crotch region of the user's undergarment can be used, but adhesive attachment means are preferred.

When adhesive attachment means 16 is present in the device, it is usually covered, prior to the time the user affixes the pantiliner to her undergarment, with release liner 17. Release liner 17 serves to keep adhesive attachment means 16 from drying out and to keep it from sticking to extraneous surfaces prior to use. Any release liner commonly used for such purposes with sanitary napkins can be used with this invention. Nonlimiting examples of suitable release liners are BL30MG-A Silox E1-0 and BL30MG-A Silox 4P/O manufactured by Akrosil Corporation.

An alternate, and especially preferred, embodiment of the present invention is illustrated as pantiliner 30 in Figure 3. Pantiliner 30 has the same plan form as pantiliner 10 illustrated in Figure 1, but it has the cross section taken along line 2—2 as illustrated in Figure 3. The difference between the embodiments illustrated in Figures 2 and 3 is the presence of wicking layer 13 interposed between overwrap 11 and resilient element 12 in pantiliner 30. Any material which causes vaginal discharges contacting the surface of pantiliner 30 to migrate along and across the under surface of top overwrap 11 thereby tending to distribute the vaginal discharges across the whole of the pantiliner can be used. One suitable technique is the provision of a layer of fibers affixed to the inner surface of the overwrap as described in the previously incorporated patent to Mulane and Smith. Preferably, wicking layer 13 comprises a sheet of tissue paper closely associated with the inner surface of top overwrap 11. Tissue papers used in commonly available facial tissue products, such as that marketed under the registered trademark Puffs by The Procter & Gamble Company of Cincinnati, Ohio can be used. Especially preferred are tissue papers manufactured by either of the processes described in U.S. Patent 3,301,746 issued to Sanford and Sisson on January 31, 1967 and U.S. Patent 3,994,771 issued to Morgan and Rich on November 30, 1976.

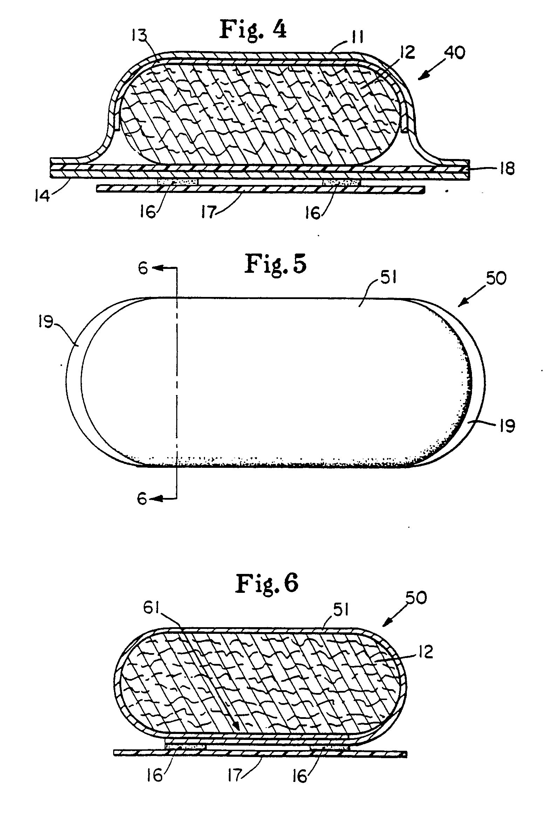

Figure 4 illustrates a second alternate embodiment of the present invention, pantiliner 40. Pantiliner 40 has the same plan form as pantiliner 10 illustrated in Figure 1, but it has the cross section taken along line 2—2 as illustrated in Figure 4. Pantiliner 40 differs from pantiliner 30 in that it possesses an optional liquid barrier 18 interposed between resilient element 12 and bottom overwrap 14. (An alternate positioning of liquid barrier 18 is on the opposite surface of bottom overwrap 14 so that bottom overwrap 14 is interposed between liquid barrier 18 and resilient element 12). In general, a liquid barrier is not needed in the pantiliner of the present invention because of the relatively small volume of vaginal discharge the device is intended to absorb. The absence of a liquid barrier allows the pantiliner to be that much smaller, less bulky, softer, more flexible, and compressible. Those skilled in the art, however, can readily select materials for liquid barrier 18 which do not significantly detract from the comfort of the present invention. Suitable materials include, for example, polyethylene film having a thickness of from 0.25 to 1.0 millimeter. Woven and nonwoven fabrics which have been treated to render them liquid repellant can also be used. Breathable, liquid resistant materials, and as those described in U.S. Patent 3,881,489 issued to Hartwell on May 6, 1975 and U.S. Patent 3,989,867 issued to Sisson on November 2, 1976 can also be used.

A still further embodiment of the present invention is illustrated in Figures 5 and 6. Pantiliner 50 has, in plan form, generally linear longitudinal edges and rounded ends while pantiliners 10, 30, and 40 are of hourglass shape with rounded ends. Construction of pantiliner 50 with essentially linear longitudinal edges allows the optional use of a single section of overwrap material as overwrap 51. Overwrap 51 passes completely about resilient element 12 and overlaps it at the rear of pantiliner 50 in the vicinity of region 61. Overwrap 51 is secured to itself in vicinity of region 61 by securement means (not shown) such as adhesive attachment. Overwrap 51 is also secured to itself at the distal ends of pantiliner 50 along end seals 19 by any convenient means such as adhesive attachment.

Preferably, the pantiliner of this invention is of hourglass shape as illustrated by pantiliner 10 in Figure 1. The size of pantiliner 10 can be conveniently selected by those skilled in the art. Typically, pantiliner 10 is from 5 to 8 centimeters wide at its widest point and is from 3 to 5 centimeters wide at its narrowest point. Typically, pantiliner 10 is from 12 to 16 centimeters from distal end to distal end. It is typically from 0.3 to 6 centimeters thick in its uncompressed state.

The absorbent capacity of resilient element 12, on an absolute basis, has been found to be not particularly critical. Obviously, a more absorbent resilient element would be preferred to a less absorbent resilient element provided the compressibility and the resilience of the two were equal. Compressibility and resilience, which directly affect the perceived comfort of the device by the user, are, then, more important in the design and construction of the present invention than is the absolute absorbent capacity of the resilient element.

Example

A pantiliner having the planform illustrated in Figure 1 and the cross-sectional configuration illustrated in Figure 3 is constructed. The top overwrap and the bottom overwrap are both formed thermoplastic films as described above. The resilient element is a latex-bonded polyester manufactured by Pellon Corporation and sold under the Pellon trademark; it is 0.4 centimeter thick (uncompressed) and weighs 1.4 grams. The overwraps are sealed one to the other by ultrasonic welding. The wicking layer comprises a single sheet of Puffs tissues as described above. The pantiliner is 14.7 centimeters long, 6.6 centimeters wide at its widest point, and 5.6 centimeters wide at its narrowest point. Adhesive attachment means comprising two longitudinal strips of Century A-3051V adhesive covered by BL3MG-A Sicox E1-0 release liner are used. In use, the pantiliner is found to be comfortable to wear, and it effectively and efficiently absorbs small amounts of vaginal discharge.

**Claims**

1. A pantiliner comprising a means (12) for containing vaginal discharges and a porous overwrap (11, 14, 51) enclosing said means, said pantiliner optionally being provided with adhesive attachment means for securement to the undergarment of the wearer, said means for containing vaginal discharges comprising a batt of hydrophilic fibers bonded together so as to provide resiliency characterised in that said batt consists solely of bonded synthetic fibers, the surfaces of said fibers being hydrophilic, said bonded fibrous batt having a density of from 0.01 g to 0.1 g/ml and being capable of recovery to 80% of its original volume after release from a compression force that reduces its volume to 20% of its original volume, said resilience being essentially unaffected by moisture.

2. A pantiliner according to Claim 1 wherein said pantiliner includes an additional component comprising a wicking layer.

3. A pantiliner according to Claim 2 which includes as an additional batt of fibers a liquid impervious sheet interposed between said resilient element and said porous overwrap.

**Patentansprüche**

1. Slipeinlage, umfassend eine Einrichtung (12) zum Aufnehmen von Vaginalausscheidungen, und eine poröse Umhüllung (11, 14, 51), von der die genannte Einrichtung umschlossen wird, wobei die genannte Slipeinlage gegebenenfalls mit Klebebefestigungsmitteln zum Befestigen an der Unterwäsche der Trägerin versehen ist, und wobei die genannte Einrichtung zum Aufnehmen von Vaginalausscheidungen ein Kissen aus hydrophilen Fasern umfaßt, welche letzteren derart miteinander verbunden sind, daß dem Kissen dadurch Rückprallelastizität verliehen wird, dadurch gekennzeichnet, daß das genannte Kissen ausschließlich aus gebundenen synthetischen Fasern besteht, wobei die Oberflächen der genannten Fasern hydrophil sind, und wobei das genannte gebundene Faserkissen eine Dichte von 0,01 g/ml bis 0,1 g/ml aufweist und befähigt ist, nach Aufhebung einer zusammendrückenden Kraft, welche das Volumen das Kissens auf 20% seines ursprünglichen Volumens verkleinert, auf 80% seines ursprünglichen Volumens zurückzufedern, wobei die genannte Rückprallelastizität von Feuchtigkeit im wesentlichen nicht beeinflußt wird.

2. Slipeinlage nach Anspruch 1, wobei die genannte Slipeinlage ein zusätzliche Komponente enthält, welche eine dochtartig aufsaugende und verteilende Schicht umfaßt.

3. Slipeinlage nach Anspruch 2, welche als zusätzliches Faserkissen ein flüssigkeitsundurchlässiges Blatt enthält, welches zwischen dem genannten elastischen Element und der genannten porösen Umhüllung eingelegt ist.

**Revendications**

1. Un protège-slip comprenant un moyen (12) pour contenir les dècharges vaginales et une enveloppe poreuse (11, 14, 51) enfermant ledit moyen, ledit protège-slip étant muni, facultativement, d'un moyen de fixation adhésif pour le fixer au sous-vêtement de l'utilisateur, ledit moyen pour contenir les décharges vaginales comprenant une motte de fibres hydrophiles reliées entre elles de manière à assurer une élasticité, caractérisé en ce que ladite motte est uniquement constituée de fibres synthétiques reliées entre elles, les surfaces desdites fibres étant hydrophiles, ladite motte de fibres reliées entre elles ayant une densité comprise entre 0,01 g et 0,1 g/ml et étant capable de revenir jusqu'à 80% de son volume initial après dégagement d'une force de compression qui réduit son volume à 20% de son volume initial, ladite élasticité résistant essentiellement à l'humidité.

2. Un protège-slip selon la revendication 1, dans lequel ledit protège-slip comporte un élément additionnel comprenant une couche à effet de mèche.

3. Un protège-slip selon la revendication 2, qui comporte en tant que motte de fibres additionnelle, une feuille imperméable aux liquides, interposée entre ledit élément élastique et ladite enveloppe poreuse.

## Fig. 1

## Fig. 2

## Fig. 3

### Fig. 4

### Fig. 5

### Fig. 6